# EUROPEAN PATENT SPECIFICATION

(11) **EP 1 088 220 B1**
(45) Date of publication and mention of the grant of the patent: **04.11.2015**
(21) Application number: 99930562.6
(22) Date of filing: 22.06.1999
(51) Int. Cl.: G01N 27/00

(54) **Polymer/plasticizer based sensor array**
Sensorarray auf Polymer/Weichmacher-Basis
Réseau de capteurs à base de polymères/plastifiant

(30) Priority: 23.06.1998 US 90412 P
(43) Date of publication of application: 04.04.2001
(73) Proprietor: CALIFORNIA INSTITUTE OF TECHNOLOGY, Pasadena, CA 91125 (US)
(72) Inventor: GRUBBS, Robert,, Pasadena, CA 91125 (US); MATZGER, Adam J.,, Pasadena, CA 91125 (US); LEWIS, Nathan S.,, Pasadena, CA 91125 (US)
(74) Representative: Bizley, Richard Edward
(86) International application number: PCT/US1999/014089
(87) International publication number: WO 1999/067627

(56) References cited:
- DE-A1- 4 018 554
- DE-A1- 19 509 518
- JP-A- 7 027 731
- US-A- 4 587 101
- US-A- 5 336 388
- US-A- 5 554 339
- US-A- 5 698 089
- BUHLMANN K ET AL: "Clathrates as coating materials for dielectric transducers with regard to organic solvent vapour sensors" SENSORS AND ACTUATORS B, ELSEVIER SEQUOIA S.A., LAUSANNE, CH, vol. 26, no. 1-3, 1995, pages 158-161, XP004318445 ISSN: 0925-4005
- R. EUGSTER ET. AL.: "Plasticizer for liquid polymeric membranes of ion-selective chemical sensors" ANALYTICA CHIMICA ACTA, vol. 289, 1994, pages 1-13, XP002473736

## Description

### FIELD OF THE INVENTION

This invention relates to a novel class of vapor sensors with tunable properties. More particularly, this invention relates to vapor sensors modified by the addition of a compatible small molecule of low volatility, *i.e.,* a plasticizer.

### BACKGROUND OF THE INVENTION

There is considerable interest in developing chemically sensitive sensors that are capable of detecting the presence of a particular chemical analyte in a fluid. Because the fluid is typically air, such sensors act as electronic noses, "smelling" the presence of a particular airborne molecule. These sensors are often fabricated from a polymeric organic material that is capable of absorbing a chemical analyte which comes in contact therewith, wherein absorbance of the analyte causes the polymeric material to expand or change, thereby modifying the electrical properties of the sensor. Variability in the ability to absorb an analyte results in variability in the detectable signal produced. Such organic polymer-based sensors have found use in a variety of different applications and devices including, for example, devices that function as analogs of the mammalian olfactory system (*see,* U.S. Patent No. 5,571,401, which issued to Lewis et al.*,* Lundström et al., Nature 352:47-50 (1991) and Shurmer and Gardner, Sens. Actuators B 8:1-11 (1992)), bulk conducting polymer films (Barker et al., Sens. Actuators B 17:143 (1994) and Gardner et al., Sens. Actuators B 18:240 (1994)), surface acoustic wave devices (Grate et al., Anal. Chem. 67:2162 (1995), Grate et al., Anal. Chem. 65:A987 (1993) and Grate et al., Anal. Chem. 65:A940 (1993)), fiber optic micromirrors (Hughes et al., J. Biochem. and Biotechnol. 41:77 (1 993)), quartz crystal microbalances (Chang et al., Anal. Chim. Acta 249:323 (1991)) and dye impregnated polymeric coatings on optical fibers (White et al., Anal. Chem. 68:2191 (1996)). To date, however, many of the sensors employed in the above-described devices have been fabricated from limited numbers of polymeric components and, therefore, are limited in the responses they are capable of producing.

Further, today's technology lags far behind the ability of canines or humans to detect or distinguish between chemical analytes. As a consequence, certain work is limited by the suitability of animals or humans to execute tasks. For example, quality control of food products can require production line employees to smell each item. Unfortunately, the ability of individuals to adequately discriminate odors diminishes after a short period of time, *e.g*., in about two hours. In addition, mammalian olfactory senses are limited in their ability to identify certain vapors. For example, water vapor is not detectable by smell. Further, mammalian olfactory senses are limited to identifying gaseous components, with no ability to identify or "smell" solutes in liquids.

Recent studies have shown that arrays of chemically sensitive sensors, such as those disclosed in U.S. Patent No. 5,571,401, formed from a library of expandable insulating organic polymers containing a conductor such as carbon black, are broadly responsive to a variety of analytes, yet allow classification and identification of organic vapors through application of pattern recognition methods. (Lonergan et al., Chem. Mater. 8:2298 (1996)). To date, these array elements have been fabricated from a relatively small number of approximately 10-20 organic polymers, with a single distinct polymer backbone composition in each sensor element. Although a limited number of polymeric sensor compositions might be chosen to perform optimally for specific applications, attempts to perform complex applications, such as to mimic the sense of olfaction, in which the sensing task is time dependent or is not defined in advance of the sensor array construction, will almost certainly require use of polymeric sensor libraries that are far more extensive and compositionally diverse than those presently known.

In general, plasticizers are organic compounds added to polymers to facilitate processing and to increase the flexibility and toughness of the polymeric product. Among the more important plasticizers are nonvolatile organic liquids and low melting solids such as phthalates, adipate and sebacate esters, polyols such as ethylene glycol and tricresyl phosphates.

U.S. Patent No. 4,948,490, which issued to Venkatasetty, discloses a single cell electrochemical sensor utilizing a conducting polymeric solid electrolyte film. These conducting polymeric films such as polyethylene oxide, polypropylene oxide and polyvinylidine fluoride can be used with a plasticizer. A preferred plasticizer is polyethylene glycol dimethyl ether. The plasticizer is added to the mixture to increase ionic conductivity by converting some or substantially all of the structure from crystalline to a plasticized amorphous form.

In addition, U.S. Patent No. 4,587,101, discloses a fluorescence oxygen sensor having a plasticized polymer with fluorescent indicator molecules embedded within the polymer. In operation, the presence of the oxygen reduces the intensity of the fluorescent indicator substance, thus facilitating detection.

European Patent Application No. 0 794 428, published September 10, 1997, describes sensors capable of distinguishing between enantiomers. The sensor comprise a pair of spaced apart contacts and a conducting polymer material spanning the gap. The polymer has chiral sites in the polymer material formed by incorporating optically active counter ions such as camphor sulfonic acid.

WO 99/00663, published January 7, 1999, discloses a sensor in which at least a first and second organic polymer are combined to form an organic polymer blend. The sensor will preferably provide a signal that is not linearly related to the mole fraction of at least one of the organic polymers used to produce the organic polymer blend.

In view of the foregoing, and despite the advances disclosed in WO 99/00663, there still remains a need for novel methods for producing large libraries of radically sensitive sensors having tunable properties, each of which are capable of producing a detectable response in the presence of an analyte of interest. The present invention fulfills this and other needs.

### SUMMARY OF THE INVENTION

In the present invention, sensor arrays as defined in claim 1 are constructed with at least a first and a second sensor wherein each sensor comprises an insulating organic polymer. The first and second sensors include a plasticizer combined with the polymer. A detector is operatively associated with each sensor. The detector is an electrical measuring device electrically coupled to the sensor to measure the first and second electrical responses.

Methods of using the sensors are also provided. One embodiment is a method for detecting the presence of an analyte in a fluid, as defined in claim 7.

A detector is operatively associated with each sensor. The detector can detect variations in, for example, electromagnetic energy, optical properties, resistance, capacitance, inductance or impedance of a combination thereof, and other physical, chemical and electrical properties that can vary in accordance with the response of the sensors. The sensors are then exposed to a fluid containing an analyte and the sensor responses measured. The sensor responses are compared to determine the presence of an analyte in the fluid.

The polymer in the sensors includes carbon black, wherein the sensor response to the presence of the analyte, is a change in a resistance associated with the sensor. In such embodiments, the detector is an electrical measuring apparatus electrically coupled to the sensors, measuring an electrical response of each sensor. These and other embodiments will become more apparent when read with the accompanying detailed description of the invention.

### DETAILED DESCRIPTION OF THE INVENTION AND PREFERRED EMBODIMENTS

In certain aspects, the present invention provides methods for producing large libraries of sensors having tunable properties, each of which are capable of producing a detectable response in the presence of an analyte of interest. In the presence of an analyte, a sensor comprising a polymer such as an organic polymer, will sorb the analyte, producing a detectable response in the polymer. A detector operatively associated with the sensor detects the response. In certain embodiments, the polymer is nonconductive and is mixed with an electrically conductive material. In certain instances, the sensor comprises nonconductive regions of organic polymer, such as insulating organic polymer, interspersed with regions of electrically conductive material. As the nonconductive region (or insulating phase) of the sensor sorb an analyte, the properties, such as resistance, of the sensor changes.

Arrays of such sensors are disclosed in U.S. Patent No. 5,571,401, which issued to Lewis, et al*.* Many different polymers can be used. Suitable insulating organic polymers include, but are not limited to, main-chain carbon polymers such as poly(dienes), poly(alkenes), poly(acrylics), poly(methacrylics), poly(vinyl ethers), poly(vinyl thioethers), poly(vinyl alcohols), poly(vinyl ketones), poly(vinyl halides), poly(vinyl nitriles), poly(vinyl esters), poly(styrenes), poly(arylenes), and the like, main-chain acrylic heteroatom organic polymers such as poly(oxides), poly(carbonates), polyesters), poly(anhydrides), poly(urethanes), poly(sulfonates), poly(siloxanes), poly(sulfides), poly(thioesters), poly(sulfones), poly(sulfonamides), poly(amides), poly(ureas), poly(phosphazenes), poly(silanes), poly(silazanes), and the like, and main-chain heterocyclic polymers such as poly(furan tetracarboxylic acid diimides), poly(benzoxazoles), poly(oxadiazoles), poly(benzothiazinaphenothiazines), poly(benzothiazoics), poly(pyrazinoquinoxalines), poly(pyromellitimides), poly(quinoxalines), poly(benzimidazoles), poly(oxindoles), poly(oxoisoindolines), poly(dioxoisoindolines), poly(triazines), poly(pyridazines), poly(piperazines), poly(pyridines), poly(piperidines), poly(triazoles), poly(pyrazoles), poly(pyrrolidines), poly(carboranes), poly(oxabicyclononanes), poly(dibenzofarans), poly(phthalides), poly(acetals), poly(anhydrides), carbohydrates, and the like. In a preferred embodiment, the polymers employed are poly(vinyl acetate) (PVA) and poly (methacrylate) (PMMA) and polystyrene (PS). Each of the above organic polymers, and the monomer units that polymerize to form these polymers, are well known to those of skill in the art.

The conductive material is carbon black

Applicants have surprisingly discovered that by combining the organic polymer with a plasticizer, the response of the sensor to a particular analyte is changed. Note that U.S. Patent No. 5,571,401 discloses the addition of a "plasticizer" to the organic polymer within its disclosed sensor array. See, *e.g.,* Column 11, Table 3. These compounds, high molecular weight polymers such as polystyrene, were "plasticizers" in the sense that they were added to the organic polymer and affected its resulting structure. However, such compounds are not normally considered plasticizers. For example, Hawley's Condensed Chemical Dictionary, 11th Ed., defines "plasticizer" as: "An organic compound added to a high molecular weight polymer both to facilitate processing and to increase the flexibility and toughness of the final product by internal modification (solvation) of the polymer molecule." High molecular weight polymers such as those disclosed in Table 3 of U.S. Pat. No. 5,557,401 would not fall within this definition, which definition is adopted herein for all following references to "plasticizer."

Suitable plasticizers for use in the present invention are set forth in claim 1.

The discovery that plasticizers affect the resulting responses in sensors containing polymers such as organic polymers leads to important advantages. For example, machine olfaction or electronic "noses" will require the use of a large number of chemically distinct organic polymeric compounds. By the addition of a plasticizer, the number of such chemically distinct organic polymeric compounds is greatly increased. For example, consider a sensor array comprising five distinct organic compounds (and thus five different sensors). With the addition of five distinct plasticizers, the number of compositionally distinct sensors is increased from five to thirty. Moreover, the number of compositionally distinct sensors can be further increased if the plasticizer is added to an organic polymer formed as a blend of a first organic polymer and a second organic polymer. Alternatively, the plasticizer can be added to an organic polymer formed from a first organic monomer and a second organic monomer.

The plasticizer can also be added to an organic polymer forming an interpenetrating network (IPN) comprising a first organic polymer and a second organic polymer formed from an organic monomer polymerized in the presence of the first organic polymer. This technique works particularly well when dealing with polymers that are imiscible in one another, where the polymers are made from monomers that are volatile. Under these conditions, the preformed polymer is used to dictate the properties (e.g., viscosity) of the polymer-monomer mixture. Thus, the polymer holds the monomer in solution. Examples of such a system are (1) polyvinyl acetate with monomer methylmethacrylate to form an IPN of pVA and pMMA, (2) pVA with monomer styrene to form an IPN of pVA and polystyrene, and (3) pVA with acrylonitrile to form an IPN of pVA and polyacrylonitrile. Each of the example compositions would be modified by the addition of an appropriate plasticizer. More than one monomer can be used where it is desired to create an IPN having one or more copolymers.

In certain instances, the sensors comprising plasticizers show an increased response rate, such as an electrical response rate in the presence of an analyte, compared to sensors without plasticizers. An increased sensor response rate is advantageous especially when performing multiple sampling because the recycle time is increased dramatically. By speeding the recovery rate of individual sensors, the sampling time is increased dramatically.

The present disclosure provides methods for the rapid construction of large libraries of sensors through combinatorial techniques. Combinatorial chemistry is a generic term that describes a series of innovative technologies that are designed to automate and simplify the selection, synthesis and fabrication of candidate polymers and plasticizers and combinations thereof into a library. The initial step of a combinatorial process is selection of compounds such as polymers and plasticizers, for inclusion in a library of compounds. A major focus in the synthesis of the library is the automation of each step of various operations. In many cases, 96-well or 384-well microtiter plates are used to dispense reagents. Automated systems are available that control temperature of the reactions, volume of reaction reagents, inertness of the reaction atmosphere, etc. In addition, highly automated sampling handling and analysis has been developed to analyze the volume of compounds in the library.

Preparation of combinatorial chemical libraries is well known to those of skill in the art. Such combinatorial chemical libraries include for instance, peptide libraries (*see, e.g.,* U.S. Patent 5,010,175, Furka, Int. J. Pept. Prot. Res. 37:487 (1991), Houghton et al., Nature, 354:84 (1991)). Peptide synthesis is by no means the only approach envisioned and intended for use with the methods of the present invention. Other chemistries for generating chemical diversity libraries can also be used. Such chemistries include, but are not limited to: peptoids (PCT Publication No WO 91/19735), encoded peptides (PCT Publication WO 93/20242), random bio-oligomers (PCT Publication WO 92/00091), benzodiazepines (U.S. Pat. No. 5,288,514), diversomers such as hydantoins, benzodiazepines and dipeptides (Hobbs, et al., Proc. Nat. Acad. Sci. USA 90:6909 (1993)), vinylogous polypeptides (Hagihara, et al., J. Amer. Chem. Soc. 114:6568 (1992)), nonpeptidal peptidomimetics with a α-D-glucose scaffolding (Hirschmann, et al., J. Amer. Chem. Soc. 114:9217 (1992)), analogous organic syntheses of small compound libraries (Chen, et al., J. Amer. Chem. Soc. 116:2661 (1994)), oligocarbamates (Cho, et al., Science 261:1303 (1993)), and/or peptidyl phosphonates (Campbell, et al., J. Org. Chem. 59:658 (1994)). See, generally, Gordon, et al., J. Med. Chem. 37:1385 (1994), nucleic acid libraries, peptide nucleic acid libraries *(see, e.g.,* U.S. Patent 5,539,083) antibody libraries *(see, e.g.,* Vaughn, et al., Nature Biotech. 14(3):309-314 (1996), and PCT/US96/10287), carbohydrate libraries *(see, e.g.,* Liang, et al., Science 274:1520 (1996), and U.S. Patent 5,593,853), and small organic molecule libraries (*see, e.g.,* benzodiazepines, Baum, C&EN, Jan 18, 1993 p. 33; isoprenoids, U.S. Patent 5,569,588; thiazolidinones and metathiazanones, U.S. Patent 5,549,974; pyrrolidines, U.S. Patents 5,525,735 and 5,519,134; morpholino compounds, U.S. Patent 5,506,337; benzodiazepines 5,288,514, cyclopentane carboxylic acid (cispentacin) compounds (Jethwaney, D., et al., Microbiology 143:397 (1997) and the like).

Devices for the preparation of combinatorial libraries are commercially available (see, e.g., 357 MPS, 390 MPS, Advanced Chem Tech, Louisville KY, Symphony, Rainin, Woburn, MA, 433A Applied Biosystems, Foster City, CA, 9050 Plus, Millipore, Bedford, MA). A number of well known robotic systems have also been developed for solution phase chemistries. These systems include automated workstations like the automated synthesis apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.) which mimic the manual synthetic operations performed by a person of skill in the art. The nature and implementation of modifications to these devices (if any) so that they can operate as discussed herein will be apparent to skilled artisans. In addition, numerous combinatorial libraries are themselves commercially available *(see, e.g.,* ComGenex, Princeton, N.J., Asinex, Moscow, Ru, Tripos, Inc., St. Louis, MO, ChemStar, Ltd, Moscow, RU, 3D Pharmaceuticals, Exton, PA, Martek Biosciences, Columbia, MD, etc.).

In other embodiments, peptide mimetics, called "peptoids", are assembled and screened for the desired biological activity by a range of methodologies (see, Gordon et al., J. Med Chem., 37: 1385-1401 (1994). For example, the method of Geysen, (Bioorg. Med. Chem. Letters, 3: 397-404 (1993); Proc. Natl. Acad Sci. USA, 81: 3998 (1984)) employs a modification of Merrifield peptide synthesis, wherein the C-terminal amino acid residues of the peptides to be synthesized are linked to solid-support particles shaped as polyethylene pins; these pins are treated individually or collectively in sequence to introduce additional amino-acid residues forming the desired peptides.

Houghton, Proc. Natl. Acad Sci. USA, 82: 5131 (1985); Eichler et al., Biochemistry, 32: 11035-11041 (1993); and U.S. Pat. No. 4,631,211) utilizes individual polyethylene bags ("tea bags") containing C-terminal amino acids bound to a solid support. These are mixed and coupled with the requisite amino acids using solid phase synthesis techniques. The peptides produced are then recovered and used individually. Fodor et al., Science, 251: 767 (1991) described light-directed, spatially addressable parallel-peptide synthesis on a silicon wafer to generate large arrays of addressable peptides that can be directly used. The particulate material of the invention can be utilized in a similar manner.

Parallel synthesis of "small" molecules (non-oligomers with a molecular weight of 200-1000) can also be used. Recently, Ellmann disclosed the solid phase-supported parallel (also referred to as "combinatorial") synthesis of eleven benzodiazepine analogs along with some prostaglandins and beta-turn mimetics. These disclosures are exemplified in U.S. Pat. No. 5,288,514. Another relevant disclosure of parallel synthesis of small molecules can be found in U.S. Pat. No. 5,324,483. This patent discloses the parallel synthesis of between 4 and 40 compounds in each of sixteen different scaffolds. Chen *et al.* have also applied organic synthetic strategies to develop non-peptide libraries synthesized using multi-step processes on a polymer support. (Chen et al., J. Am. Chem. Soc., 116: 2661-2662 (1994)).

Plasticizer polymer sensors provide superior properties as compared to an unmodified organic polymer because of the lowered glass transition temperature in the plasticized organic polymer. Also, the plasticized polymer have superior processing properties, enhanced film stability, increased response rates, lower usable temperature range and greater chemical stability than the unmodified organic polymer. Finally, the plasticizer allow sensor fabrication without the use of volatile solvents because of the superior melt processing properties of the plasticized polymers.

A broad range of analytes can be detected using the sensors of the present invention. Suitable analytes include, but are not limited to, alkanes, alkenes, alkynes, dienes, alicyclic hydrocarbons, arenes, alcohols, ethers, ketones, aldehydes, carbonyls, carbanions, heterocycles, polynuclear aromatics, organic derivatives, biomolecules, microorganisms, bacteria, viruses, sugars, nucleic acids, isoprenes, isoprenoids, fatty acids and their derivatives. In certain embodiments, many biomolecules, such as amino acids, are amenable to detection using the sensor arrays of the present invention.

The sensors of the present invention can be used for a variety of applications. Suitable applications include, but are not limited to, environmental toxicology and remediation, biomedicine, such as microorganism classification or detection, medical diagnosis, material quality control, food and agricultural products monitoring, heavy industrial manufacturing, ambient air monitoring, worker protection, emissions control, and product quality testing.

Various sensors suitable for use in the present invention include, but are not limited to, conducting/nonconducting sensors, bulk conducting polymer films, surface acoustic wave devices, fiber optic micromirrors, quartz crystal microbalances, dye impregnated polymeric coatings on optical fibers, sintered metal oxide sensors, phthalocyanine sensors, Pd-gate MOSFET devices, electrochemical cells, conducting polymer sensors, lipid coating sensors, metal FET structures, carbon black-polymer composites, micro-electro-mechanical system devices, micromachined cantilevers, and a micro-opto-electro-mechanical system devices.

Moreover, various analyses suitable for identifying analytes and quantifying concentration include, but are not limited to, principal component analysis, Fischer linear analysis, neural networks, genetic algorithms, fuzzy logic, pattern recognition, and other algorithms. After analysis is completed, the resulting information is displayed or transmitted to a host computer.

In accordance with the invention, a sensor array was formed as follows. The organic polymer comprised a polymer selected from the group consisting of poly(methyl methacrylate) (PMMA), polystyrene (PS), and poly(vinylchloride) (PVC). The organic polymer was combined with carbon black. A plasticizer chosen from the group consisting of di(2-ethylhexyl)phthalate (DOP), diethylene glycol dibenzoate (DGD), glycerol triacetate (GT), tributyl phosphate (TBP), chloroparafin (50% Cl, CP), and tricresyl phosphate (TCP) was added to the organic polymer.

Glass substrates for sensor production were prepared by evaporating 300 Å of chromium followed by 500 Å of gold onto microscope slides masked with a 3 mm strip of drafting tape down the center of the long axis. These were then cut down their short axis to give 15 smaller pieces. Individual sensor elements for PMMA and PVC sensors were prepared by spin coating from a solution of the organic polymer, an appropriate solvent, the plasticizer, and suspended carbon black onto the glass substrates. The electrodes and backs of the slides were cleaned with solvent prior to using the sensors. Five replicates of each sensor were made for each experiment. PS sensors were prepared on surface mount universal boards (surfboards, part 6012 from Capital Advanced Technologies) by dip coating into a solution of the organic polymer, an appropriate solvent, the plasticizer, and suspended carbon black. Those of ordinary skill in the art will appreciate that possible sensor fabrication techniques include spin coating, dip coating, spray coating, and evaporation of a droplet. The following table, Table 1, sets forth the composition of the solutions that were used to either spin or dip coat the sensor substrates. The carbon black used in these solutions was Black Pearls 2000, a furnace material produced by Cabot Co. (Billerica, MA). Poly(methyl methacrylate) (PMMA, mw 120,000) and polystyrene (PS, mw 45,000) were purchased from Aldrich Chemical Co.(Milwaukee, WI). Poly(vinylchloride) (PVC, raw 275,000) and all plasticizers were obtained from Polysciences, Inc. (Warrington, PA).

**Table 1**

| Solution | Polymer/ Solvent | Polymer (mg) | Carbon black(mg) | Plasticizer | Plasticizer (mg) |
|---|---|---|---|---|---|
| A | PMMA/THF | 161 | 39.6 | none | - |
| B | PMMA/THF | 161 | 40.7 | DOP | 39.2 |
| C | PMMA/THF | 161 | 40.3 | DGD | 46.6 |
| D | PMMA/THF | 161 | 40.9 | GT | 38.2 |
| E | PMMA/THF | 161 | 40.5 | TBP | 38.9 |
| F | PMMA/THF | 161 | 40.5 | TBP | 9.4 |
| G | PMMA/THF | 161 | 41.2 | TBP | 18.1 |
| H | PMMA/THF | 161 | 41.1 | TBP | 75.6 |
| I | PS/benzene | 162 | 42.7 | none | - |
| J | PS/benzene | 162 | 40.9 | DGD | 39.0 |
| K | PS/benzene | 162 | 44.5 | CP | 39.3 |
| L | PS/benzene | 162 | 42.6 | TBP | 42.6 |
| M | PS/benzene | 162 | 43.9 | DOP | 43.7 |
| N | PS/benzene | 162 | 41.0 | DOP | 10.0 |
| O | PS/benzene | 162 | 40.0 | DOP | 21.0 |
| P | PS/benzene | 162 | 42.5 | DOP | 80.7 |
| Q | PVC/THF | 160 | 41.0 | none | - |
| R | PVC/THF | 160 | 39.8 | DOP | 40.8 |
| S | PVC/THF | 160 | 40.5 | DGD | 39.71 |
| T | PVC/THF | 160 | 40.4 | TCP | 39.6 |

| | | | | | |
|---|---|---|---|---|---|
| Note that a letter identified each individual solution. | | | | | |

The sensors prepared from the above solutions were exposed to an analyte, which in this case was the vapor of a number of common solvents. The solvents used as the chemical analyte were hexane, toluene, chloroform, tetrahydrofuran (THF), acetone, ethyl acetate, ethanol, and methanol. In each case, the vapor was diluted to 5% of its vapor pressure. PMMA- and PVC-based sensors were exposed 15 times to each analyte. PS-based sensors were exposed 5 times to each analyte. The analytes were presented in random order. An apparatus for delivering known concentrations of organic vapors was constructed from solvent bubblers, solenoids, and mass flow controllers to precisely control the concentration of the analyte delivered to each sensor. Flow switching and data acquisitions were computer controlled with Lab VIEW software.

Sensor resistance measurements as a function of time were recorded before, during, and after analyte exposure using a Keithly model 2002 multimeter attached to a Keithly model 7001 channel switcher. The data were analyzed for the magnitude of the response by subtracting the median of the of the baseline resistance (before analyte exposure) from the median of the resistance (taken at the end of the analyte exposure) and dividing by the baseline resistance. This ΔR/R was the unique quantity measured for a given sensor during each exposure.

Sensor responses to various solvents were analyzed through the use of a Fisher linear discriminant method. This method provides a measure of the resolving power of a given sensor array for the set of solvents. The result is a matrix of resolution factors describing the ability of the array to distinguish between pairs of solvents.

The effect of added plasticizer is illustrated below in detail for PVC. The results are summarized in tables for each solution that contained PVC, namely solutions Q through T.

**Solution Q**

| | Toluene | Chloroform | THF | Acetone | Ethyl Acetate | Ethanol | Methanol |
|---|---|---|---|---|---|---|---|
| Hexane | 0.984251 | 0.643293 | 0.842919 | 1.28274 | 0.84445 | 1.53139 | 4.61927 |
| Toluene | | 0.658509 | 0.807142 | 2.06814 | 1.07682 | 2.4402 | 5.06123 |
| Chloroform | | | 0.665612 | 1.4629 | 0.587399 | 1.91321 | 4.9815 |
| THF | | | | 1.45015 | 0.774263 | 1.885012 | 4.91201 |
| Acetone | | | | | 0.847823 | 0.282196 | 3.98495 |
| Ethyl Acetate | | | | | | 1.19 | 4.484 |
| Ethanol | | | | | | | 4.19594 |

**Solution R**

| | Toluene | Chloroform | THF | Acetone | Ethyl Acetate | Ethanol | Methanol |
|---|---|---|---|---|---|---|---|
| Hexane | 4.07487 | 6.50677 | 6.26755 | 12.0878 | 8.62828 | 5.01794 | 7.96911 |
| Toluene | | 3.37885 | 3.63363 | 6.93624 | 4.22286 | 2.44464 | 6.99162 |
| Chloroform | | | 1.0114 | 3.80078 | 0.552501 | 5.14984 | 8.84274 |
| THF | | | | 3.31752 | 1.03253 | 4.57039 | 7.50424 |
| Acetone | | | | | 4.30155 | 8.64767 | 12.0074 |
| Ethyl Acetate | | | | | | 6.12712 | 10.0452 |
| Ethanol | | | | | | | 6.78787 |

**Solution S**

| | Toluene | Chloroform | THF | Acetone | Ethyl Acetate | Ethanol | Methanol |
|---|---|---|---|---|---|---|---|
| Hexane | 0.407902 | 1.45887 | 1.6386 | 4.64552 | 1.66585 | 3.14121 | 4.81276 |
| Toluene | | 1.31997 | 1.24821 | 4.48867 | 1.44003 | 2.87767 | 5.04127 |
| Chloroform | | | 0.532754 | 2.76737 | 0.670284 | 1.54771 | 4.52271 |
| THF | | | | 3.98235 | 0.577512 | 2.30406 | 4.55185 |
| Acetone | | | | | 3.67577 | 1.52751 | 2.65537 |
| Ethyl Acetate | | | | | | 2.10352 | 4.24966 |
| Ethanol | | | | | | | 3.6201 |

**Solution T**

| | Toluene | Chloroform | THF | Acetone | Ethyl Acetate | Ethanol | Methanol |
|---|---|---|---|---|---|---|---|
| Hexane | 3.37051 | 4.88942 | 6.08152 | 9.06222 | 8.40895 | 7.68271 | 9.7596 |
| Toluene | | 3.44704 | 3.55683 | 7.66181 | 4.70103 | 4.56691 | 6.78456 |
| Chloroform | | | 0.721996 | 5.07964 | 0.406391 | 2.66707 | 5.84979 |
| THF | | | | 6.35071 | 0.84786 | 2.71799 | 5.1942 |
| Acetone | | | | | 6.38207 | 7.5861 | 7.48886 |
| Ethyl Acetate | | | | | | 3.52548 | 5.50609 |
| Ethanol | | | | | | | 4.94153 |

The average resolution factor for unplasticized PVC (solution Q) is 2.02. The corresponding numbers for solutions R, S, and T are 2.62, 5.19, and 5.78, respectively. By combining all of the sensors, to make an array with five sensors each from solutions Q-T provides an average resolution factor of 13.11, as demonstrated in the following table.

**Solutions Q-T**

| | Toluene | Chloroform | THF | Acetone | Ethyl Acetate | Ethanol | Methanol |
|---|---|---|---|---|---|---|---|
| Hexane | 7.42856 | 12.7416 | 13.2806 | 22.0313 | 14.7446 | 10.1892 | 24.025 |
| Toluene | | 6.10203 | 9.71362 | 12.5442 | 9.43622 | 10.4272 | 15.6674 |
| Chloroform | | | 4.49606 | 8.27175 | 6.07853 | 9.70826 | 19.1793 |
| THF | | | | 10.7487 | 4.27985 | 11.0709 | 18.3842 |
| Acetone | | | | | 10.9168 | 16.8513 | 23.9641 |
| Ethyl Acetate | | | | | | 12.3627 | 22.4903 |
| Ethanol | | | | | | | 19.9709 |

The benefits of the present invention are also seen for the PA-based sensor (formed from solutions A-H) and are summarized in the following Table 2.

**Table 2**

| Solution resolution factor | Maximum resolution factor | Minimum resolution factor | Average resolution factor |
|---|---|---|---|
| A | 14.94 | 1.11 | 5.55 |
| B | 18.69 | 1.6 | 9.23 |
| C | 21.26 | 2.74 | 11.37 |
| D | 12.59 | 0.89 | 5.35 |
| E | 16.4 | 0.82 | 6.88 |
| F | 17.7 | 1.67 | 7.78 |
| G | 17.02 | 2.61 | 9.24 |
| H | 19.33 | 1.85 | 7.04 |
| A-D | 78.41 | 9.75 | 32.88 |
| E-H | 59.22 | 7.48 | 7.48 |

Five presentations of each solvent were made for the polystyrene sensors, and qualitative comparison shows that the magnitude of the responses can be affected by the presence of various plasticizers. Sensors made from the plasticized polymer were more likely to be responsive to solvent.

Those of ordinary skill in the art will appreciate that many other embodiments of the invention could be implemented. For example, the concentration of a given plasticizer in a given organic polymer can be varied across a sensor array. Assuming that the sensor response is non-linearly related to the varying plasticizer concentration, additional chemical analyte resolving power will be provided without requiring the use of additional organic polymers or plasticizers. Similarly, the plasticized polymer could be formed from the addition of a plasticizer to an organic polymer blend of a first organic polymer and a second organic polymer. The relative concentration of the first and second organic polymer could be varied in the organic polymer blend, and, assuming a nonlinear sensor response, the resulting sensor array would have greater resolving power.

Many other embodiments exist for sensors that need not include the electrically conductive material with organic polymer and plasticizer mixture. Such sensors can be associated with detectors that measure a response other than the electrical resistance change upon exposure to the chemical analyte. For example, the detector can measure variations in optical transmission through the sensor wherein the detector would be a spectrophotometer. Alternatively, the detectable response is a variation in electromagnetic energy, and the detector measure electromagnetic energy.

Although the invention has been described with reference to preferred embodiments and examples thereof, the scope of the present invention is not limited only to those described embodiments. As will be apparent to persons skilled in the art, modifications and adaptations to the above-described invention can be made without departing from the scope of the invention, which is defined and circumscribed by the appended claims.

## Claims

1. A sensor array for detecting an analyte in a fluid, the sensor array comprising at least a first and a second sensor, each of said first and second sensors comprising an insulating organic polymer mixed with both a plasticizer and an electrically conductive material and a detector operatively associated with each of the first and second sensors, wherein the detector is an electrical measuring device electrically coupled to the first or second sensors so as to measure first and second electrical resistances; said measuring devices of said array being operatively coupled to a computer with a resident algorithm; wherein said insulating organic polymer is a member selected from the group consisting of poly(methyl methacrylate) (PMMA), polystyrene (PS), and poly(vinylchloride) (PVC), and wherein said plasticizer is a member selected from the group consisting of di(2-ethylhexyl)phthalate (DOP), diethylene glycol dibenzoate (DGD), glycerol triacetate (GT), tributyl phosphate (TBP), chloroparafin (50% Cl, CP), and tricresyl phosphate (TCP) and said electrically conductive material is carbon black.

2. A sensor array in accordance with claim 1, wherein either:
(a) said insulating organic polymer is formed from a blend of a first organic polymer and a second organic polymer; or
(b) said insulating organic polymer is formed from a first organic monomer and a second organic monomer; or
(c) said insulating organic polymer is formed from an interpenetrating network comprising a first organic polymer and a second organic polymer formed from an organic monomer polymerized in the presence of the first organic polymer.

3. A sensor array in accordance with claim 1, wherein said algorithm of said computer is a member selected from the group consisting of principal component analysis, Fischer linear analysis, neural networks, genetic algorithms, fuzzy logic, pattern recognition, and combinations thereof.

4. A sensor array in accordance with claim 1, wherein said electric detector detects a member selected from the group consisting of electromagnetic energy, optical properties, resistance, capacitance, inductance, impedance and combinations thereof.

5. A sensor array in accordance with claim 1, wherein each of said sensors further comprises an electrically conductive material and wherein said electric detector is an electrical measuring device.

6. A sensor array in accordance with claim 1, wherein at least one of said sensors in said array is a member selected from the group consisting of conducting/nonconducting sensors, bulk conducting polymer films, surface acoustic wave devices, fiber optic micromirrors, quartz crystal microbalances, dye impregnated polymeric coatings on optical fibers, sintered metal oxide sensors, phthalocyanine sensors, Pd-gate MOSFET devices, electrochemical cells, conducting polymer sensors, lipid coating sensors, metal FET structures, carbon black-polymer composites, micro-electro-mechanical system devices, micromachined cantilevers, and micro-opto-electro-mechanical system devices.

7. A method for detecting the presence of an analyte in a fluid, said method comprising; providing a sensor array as set forth in claim 1 and contacting said array with said analyte to generate an electrical response, detecting said electrical response with said electric detector that is electrically operatively coupled to each sensor and correlating said electrical response to a known response with said computer and thereby detecting the presence of said analyte.

8. A method in accordance with claim 7, wherein said analyte is detected in an application which is a member selected from the group consisting of environmental toxicology, remediation, biomedicine, material quality control, food and agricultural products monitoring, heavy industrial manufacturing, ambient air monitoring, worker protection, emissions control, and product quality testing.

9. A method in accordance with claim 7, wherein said array is as set forth in claim 2.

10. A method in accordance with claim 7 wherein said array is as set forth in claim 3.

11. A method in accordance with claim 7, wherein at least one of said sensors in said sensor array is a member selected from the group consisting of conducting/nonconducting sensors, bulk conducting polymer films, surface acoustic wave devices, fiber optic micromirrors, quartz crystal microbalances, dye impregnated polymeric coatings on optical fibers, sintered metal oxide sensors, phthalocyanine sensors, Pd-gate MOSFET devices, electrochemical cells, conducting polymer sensors, lipid coating sensors, metal FET structures, carbon black-polymer composites, micro-electro-mechanical system devices, micromachined cantilevers, and micro-opto-electro-mechanical system devices.

12. A method in accordance with claim 7, wherein said detector is optimized to detect a member selected from the group consisting of electromagnetic energy, optical properties, resistance, capacitance, inductance, impedance and combinations thereof.

## Patentansprüche

1. Sensoranordnung zum Erkennen eines Analyten in einem Fluid, wobei die Sensoranordnung wenigstens einen ersten und einen zweiten Sensor umfasst, wobei jeder des ersten und des zweiten Sensors ein isolierendes organisches Polymer umfasst, das mit einem Weichmacher und einem elektrisch leitenden Material vermischt ist und wobei ein Detektor funktionsmäßig mit jedem des ersten und des zweiten Sensors verknüpft ist, wobei der Detektor ein elektrisches Messgerät ist, das elektrisch mit dem ersten oder dem zweiten Sensor gekoppelt ist, um einen ersten und einen zweiten elektrischen Widerstand zu messen; wobei die Messgeräte der Anordnung funktionsmäßig mit einem Computer mit einem residenten Algorithmus gekoppelt sind, wobei das isolierende organische Polymer aus der Gruppe ausgewählt ist, die aus Poly(methylmethacrylat) (PMMA), Polystyrol (PS) und Poly(vinylchlorid) (PVC) besteht, und der Weichmacher aus der Gruppe ausgewählt ist, die aus di(2-Ethylhexyl)phthalat (DOP), Diethylenglycoldibenzoat (DGD), Glyceroltriacetat (GT), Tributylphosphat (TBP), Chloroparaffin (50 % Cl, CP) und Tricresylphosphat (TCP) besteht und das elektrisch leitende Material Ruß ist.

2. Sensoranordnung nach Anspruch 1, wobei entweder:
(a) das isolierende organische Polymer aus einer Mischung aus einem ersten organischen Polymer und einem zweiten organischen Polymer gebildet ist; oder
(b) das isolierende organische Polymer aus einem ersten organischen Monomer und einem zweiten organischen Monomer gebildet ist; oder
(c) das isolierende organische Polymer aus einem interpenetrierenden Netzwerk gebildet ist, umfassend ein erstes organisches Polymer und ein zweites organisches Polymer, gebildet aus einem organischen Monomer, das in der Gegenwart des ersten organischen Polymers polymerisiert ist.

3. Sensoranordnung nach Anspruch 1, wobei der Algorithmus des Computers aus der Gruppe ausgewählt ist, die aus Hauptkomponentenanalyse, linearer Analyse nach Fischer, neuronalen Netzen, genetischen Algorithmen, Fuzzylogik, Mustererkennung und Kombinationen aus diesen besteht.

4. Sensoranordnung nach Anspruch 1, wobei der elektrische Detektor eines aus der Gruppe detektiert, die aus elektromagnetischer Energie, optischen Eigenschaften, Widerstand, Kapazität, Induktanz, Impedanz und Kombinationen aus diesen besteht.

5. Sensoranordnung nach Anspruch 1, wobei jeder der Sensoren ferner ein elektrisch leitfähiges Material umfasst und wobei der elektrische Detektor ein elektrisches Messgerät ist.

6. Sensoranordnung nach Anspruch 1, wobei wenigstens einer der Sensoren in der Anordnung aus der Gruppe ausgewählt ist, die aus leitfähigen/nicht leitfähigen Sensoren, leitfähigen Blockpolymerfilmen, akustischen Oberflächenwellengeräten, Faseroptik-Mikrospiegeln, Quarzmikrowaagen, farbimprägnierten Polymerbeschichtungen auf optischen Fasern, gesinterten Metalloxidsensoren, Phthalocyaninsensoren, Pd-Gate-MOSFET-Geräten, elektrochemischen Zellen, leitfähigen Polymersensoren, lipidbeschichteten Sensoren, Metall-FET-Anordnungen, Ruß-Polymer-Verbundstoffen, elektromechanischen Mikrosystemgeräten, mikromaterialbearbeiteten Auslegern und mikrooptoelektromechanischen Systemgeräten besteht.

7. Verfahren zum Detektieren des Vorliegens eines Analyten in einem Fluid, wobei das Verfahren Folgendes umfasst: Bereitstellen einer Sensoranordnung nach Anspruch 1 und Inberührungbringen der Anordnung mit dem Analyten, um eine elektrische Reaktion hervorzurufen, Detektieren der elektrischen Reaktion mit dem elektrischen Detektor, der funktionsmäßig elektrisch mit jedem Sensor gekoppelt ist und Korrelieren der elektrischen Reaktion mit einer bekannten Reaktion durch den Computer und somit Detektieren des Vorliegens des Analyten.

8. Verfahren nach Anspruch 7, wobei der Analyt in einer Anwendung detektiert wird, die ausgewählt ist aus der Gruppe bestehend aus Umwelttoxikologie, Sanierung, Biomedizin, Materialqualitätsprüfung, Überwachung von Nahrungsmitteln und Agrarprodukten, Schwerindustriefertigung, Raumluftüberwachung, Arbeitsschutz, Emissionskontrolle und Produktqualitätsprüfung.

9. Verfahren nach Anspruch 7, wobei die Anordnung dem Anspruch 2 entspricht.

10. Verfahren nach Anspruch 7 wobei die Anordnung dem Anspruch 3 entspricht.

11. Verfahren nach Anspruch 7, wobei wenigstens einer der Sensoren in der Sensoranordnung aus der Gruppe ausgewählt ist, die aus leitfähigen/nicht leitfähigen Sensoren, leitfähigen Blockpolymerfilmen, akustischen Oberflächenwellengeräten, Faseroptik-Mikrospiegeln, Quarzmikrowaagen, farbimprägnierten Polymerbeschichtungen auf optischen Fasern, gesinterten Metalloxidsensoren, Phthalocyaninsensoren, Pd-Gate-MOSFET-Geräten, elektrochemischen Zellen, leitfähigen Polymersensoren, lipidbeschichteten Sensoren, Metall-FET-Anordnungen, Ruß-Polymer-Verbundstoffen, elektromechanischen Mikrosystemgeräten, mikromaterialbearbeiteten Auslegern und mikrooptoelektromechanischen Systemgeräten besteht.

12. Verfahren nach Anspruch 7, wobei der Detektor optimiert ist eines aus der Gruppe zu detektieren, die aus elektromagnetischer Energie, optischen Eigenschaften, Widerstand, Kapazität, Induktanz, Impedanz und Kombinationen aus diesen besteht.

## Revendications

1. Réseau de capteurs pour détecter un analyte dans un fluide, le réseau de capteurs comprenant au moins un premier et un deuxième capteur, chacun desdits premier et deuxième capteurs comprenant un polymère organique isolant mélangé avec à la fois un plastifiant et un matériau électriquement conducteur et un détecteur associé fonctionnellement à chacun desdits premier et deuxième capteurs, dans lequel le détecteur est un dispositif de mesure électrique couplé électriquement au premier ou au deuxième capteur de manière à mesurer des première et deuxième résistances électriques ; lesdits dispositifs de mesure dudit réseau étant couplés fonctionnellement à un ordinateur avec un algorithme résident ; dans lequel ledit polymère organique isolant est un élément sélectionné dans le groupe constitué par le polyméthacrylate de méthyle (PMMA), le polystyrène (PS) et le polychlorure de vinyle (PVC), et dans lequel ledit plastifiant est un élément sélectionné dans le groupe constitué par le phtalate de di(2-éthylhexyle) (DOP), le dibenzoate de diéthylène glycol (DGD), le triacétate de glycérol (GT), le phosphate de tributyle (TBP), la chloroparaffine (CI à 50 %, CP) et le phosphate de tricrésyle (TCP) et ledit matériau électriquement conducteur est le noir de carbone.

2. Réseau de capteurs selon la revendication 1, dans lequel soit :
(a) ledit polymère organique isolant est formé d'un mélange d'un premier polymère organique et d'un deuxième polymère organique ; ou
(b) ledit polymère organique isolant est formé d'un premier monomère organique et d'un deuxième monomère organique ; ou
(c) ledit polymère organique isolant est formé d'un réseau interpénétrant comprenant un premier polymère organique et un deuxième polymère organique formé d'un monomère organique polymérisé en présence du premier polymère organique.

3. Réseau de capteurs selon la revendication 1, dans lequel ledit algorithme dudit ordinateur est un élément sélectionné dans le groupe constitué par l'analyse des composants principaux, l'analyse linéaire de Fisher, les réseaux neuronaux, les algorithmes génétiques, la logique floue, la reconnaissance des formes et leurs combinaisons.

4. Réseau de capteurs selon la revendication 1, dans lequel ledit détecteur électrique détecte un élément sélectionné dans le groupe constitué par l'énergie électromagnétique, les propriétés optiques, la résistance, la capacité, l'inductance, l'impédance et leurs combinaisons.

5. Réseau de capteurs selon la revendication 1, dans lequel chacun desdits capteurs comprend en outre un matériau électriquement conducteur et dans lequel ledit détecteur électrique est un dispositif de mesure électrique.

6. Réseau de capteurs selon la revendication 1, dans lequel au moins l'un desdits capteurs dans ledit réseau est un élément sélectionné dans le groupe constitué par les capteurs conducteurs/non conducteurs, les films polymères à conductance interne, les dispositifs à ondes acoustiques de surface, les micromiroirs optiques à fibre, les microbalances à cristal de quartz, les revêtements polymères imprégnés de colorants sur des fibres optiques, les capteurs à oxyde de métal fritté, les capteurs à phtalocyanine, les dispositifs MOSFET à grille au Pd, les cellules électrochimiques, les capteurs polymères conducteurs, les capteurs à revêtement lipidique, les structures FET métalliques, les composites noir de carbone-polymère, les dispositifs à microsystème électromécanique, les cantilevers micro-usinés et les dispositifs à microsystème optoélectromécanique.

7. Procédé de détection de la présence d'un analyte dans un fluide, ledit procédé comprenant : la fourniture d'un réseau de capteurs tel que présenté dans la revendication 1 et la mise en contact dudit réseau avec ledit analyte pour générer une réponse électrique, la détection de ladite réponse électrique avec ledit détecteur électrique qui est couplé électriquement et fonctionnellement à chaque capteur et la corrélation de ladite réponse électrique à une réponse connue avec ledit ordinateur et ainsi la détection de la présence dudit analyte.

8. Procédé selon la revendication 7, dans lequel ledit analyte est détecté dans une application qui est un élément sélectionné dans le groupe constitué par la toxicologie environnementale, la remédiation, la biomédecine, le contrôle qualité des matériaux, la surveillance des produits alimentaires et agricoles, la production industrielle lourde, la surveillance de l'air ambiant, la protection des travailleurs, le contrôle des émissions et le contrôle de la qualité des produits.

9. Procédé selon la revendication 7, dans lequel ledit réseau est tel que présenté dans la revendication 2.

10. Procédé selon la revendication 7, dans lequel ledit réseau est tel que présenté dans la revendication 3.

11. Procédé selon la revendication 7, dans lequel au moins l'un desdits capteurs dans ledit réseau de capteurs est un élément sélectionné dans le groupe constitué par les capteurs conducteurs/non conducteurs, les films polymères à conductance interne, les dispositifs à ondes acoustiques de surface, les micromiroirs optiques à fibre, les microbalances à cristal de quartz, les revêtements polymères imprégnés de colorants sur des fibres optiques, les capteurs à oxyde de métal fritté, les capteurs à phtalocyanine, les dispositifs MOSFET à grille au Pd, les cellules électrochimiques, les capteurs polymères conducteurs, les capteurs à revêtement lipidique, les structures FET métalliques, les composites noir de carbone-polymère, les dispositifs à microsystème électromécanique, les cantilevers micro-usinés et les dispositifs à microsystème optoélectromécanique.

12. Procédé selon la revendication 7, dans lequel ledit détecteur est optimisé pour détecter un élément sélectionné dans le groupe constitué par l'énergie électromagnétique, les propriétés optiques, la résistance, la capacité, l'inductance, l'impédance et leurs combinaisons.
